# EUROPÄISCHE PATENTANMELDUNG

(11) **EP 3 696 150 A1**
(43) Veröffentlichungstag der Anmeldung: **19.08.2020**
(21) Anmeldenummer: 19157306.2
(22) Anmeldetag: 14.02.2019
(51) Int. Cl.: C03C 3/095, C03C 3/087, C03C 3/062, A61K 6/02, A61K 6/027, A61C 13/00

(54) **FLUORESZIERENDE GLASKERAMIKEN UND GLÄSER MIT GEHALT AN EUROPIUM**

(71) Anmelder: Ivoclar Vivadent AG, 9494 Schaan (LI)
(72) Erfinder: Dittmer, Marc, 6800 Feldkirch (AT); Hengst, Ronny, 9470 Buchs (CH); Ritzberger, Christian, 9472 Grabs (CH)
(74) Vertreter: Uexküll & Stolberg

(57) **Zusammenfassung**

Die Erfindung betrifft Glaskeramiken und Gläser mit Gehalt an Europium, die die folgenden Komponenten enthalten:

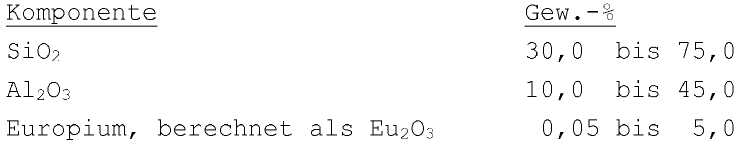

und sich insbesondere zur Herstellung von Dentalrestaurationen eignen, deren Fluoreszenzeigenschaften weitgehend denen der natürlichen Zähne entsprechen.

## Beschreibung

Die vorliegende Erfindung betrifft Glaskeramiken und Gläser, die Europium enthalten und sich insbesondere zur Herstellung von Dentalrestaurationen eignen, deren Fluoreszenzeigenschaften weitgehend denen der natürlichen Zähne entsprechen. Die Erfindung betrifft auch ein Verfahren zur Herstellung der erfindungsgemäßen Glaskeramiken und Gläser sowie deren Verwendung als Dentalmaterial und insbesondere zur Herstellung dentaler Restaurationen.

Glaskeramiken werden in der Zahnheilkunde aufgrund ihrer guten mechanischen und optischen Eigenschaften insbesondere zur Herstellung von Dentalkronen und kleinen Brücken eingesetzt.

Aus W. Buchalla, "Comparative Fluorescence Spectroscopy Shows Differences in Non-Cavitated Enamel Lesions", Caries Res. 2005, 39, 150-156 ist bekannt, dass natürliche Zähne unter ultraviolettem Licht eine bläulich-weiße Fluoreszenz mit Wellenlängen im Bereich von 400 bis 650 nm zeigen.

Rukmani et al., J. Am. Ceram. Soc. 2007, 90, 706-711, beschreiben den Einfluss von V- und Mn-Färbemitteln auf das Kristallisationsverhalten und die optischen Eigenschaften von Ce-dotierten Lithiumdisilikat-Glaskeramiken. Zur Herstellung der Glaskeramiken wird eine Mischung aus den Ausgangsmaterialien SiO₂, ZrO₂, Li₂CO₃, K₂CO₃, MgCO₃ und Al(PO₃)₃ mit CeO₂, V₂O₅ und MnO₂ hergestellt, die Mischung bei 1500°C in Platintiegeln geschmolzen, abgekühlt und anschließend in einem Röhrenofen unter Luftzufuhr mehreren Wärmebehandlungen unterzogen.

EP 0 877 071 A1 beschreibt Gläser und Glaskeramiken, die unter anderem Eu²⁺-Ionen enthalten und langanhaltende Phosphoreszenz zeigen.

DE 10 2009 013 377 A1 beschreibt die Verwendung eines Borosilikatglases, das mit CeO₂ oder mindestens einem Oxid eines anderen Lanthanoids, unter anderem Eu₂O₃, dotiert ist, zur Erhöhung der Fälschungssicherheit einer Verpackung durch eine Emission elektromagnetischer Strahlung im Bereich zwischen 300 und 700 nm bei Bestrahlung mit UV-Licht.

WO 2015/173230 A1 beschreibt ein Verfahren zur Herstellung eines Lithiumsilikatglases oder einer Lithiumsilikat-Glaskeramik, bei dem eine Schmelze eines Ausgangsglases, das Cer-Ionen enthält, reduzierenden Bedingungen ausgesetzt wird. Dadurch sollen in dem Ausgangsglas enthaltene Ce⁴⁺-Ionen ganz oder teilweise zu Ce³⁺-Ionen reduziert werden, die aufgrund von 5d→4f-Übergängen eine Fluoreszenz im Wellenlängenbereich von 320 bis 500 nm zeigen. Ein entsprechendes Verfahren zur Herstellung einer Glaskeramik mit SiO₂ als Hauptkristallphase oder eines Glases, das Keime für die Kristallisation von SiO₂ enthält, ist aus WO 2017/080853 A1 bekannt. Es hat sich jedoch gezeigt, dass die aus dem Stand der Technik bekannten Gläser und Glaskeramiken unzureichende Fluoreszenzeigenschaften aufweisen und insbesondere unter UV-Licht die Fluoreszenzeigenschaften des natürlichen Zahnmaterials nicht in ausreichendem Maße imitieren können. Insbesondere zeigen die bekannten Materialien nicht bei allen relevanten Wellenlängen im UV-Bereich die erforderliche Fluoreszenz. Dadurch werden aus solchen Glaskeramiken hergestellte Dentalrestaurationen insbesondere unter dem Einfluss von UV-Licht als Restaurationen erkennbar oder werden als Zahnlücken oder Defekte wahrgenommen. Außerdem kommt es bei den so hergestellten Gläsern und Glaskeramiken durch Wärmebehandlungen unter oxidierenden Bedingungen, beispielsweise beim Sintern, zu einer erheblichen Beeinträchtigung der Fluoreszenzeigenschaften.

Der Erfindung liegt die Aufgabe zugrunde, Glaskeramiken und Gläser bereitzustellen, die bei Anregungswellenlängen im gesamten relevanten UV-Bereich, vor allem im Bereich von 250 nm bis 430 nm und insbesondere im Bereich von 360 nm bis 430 nm, Fluoreszenz zeigen und sich somit insbesondere zur Herstellung von Dentalrestaurationen eignen, die nicht nur gute mechanische Eigenschaften aufweisen, sondern auch die Fluoreszenzeigenschaften des natürlichen Zahnmaterials bei Anregungswellenlängen im gesamten relevanten UV-Bereich weitgehend imitieren können. Insbesondere sollen sich die Glaskeramiken und Gläser auch als Mischungskomponenten zur Einstellung der Fluoreszenzeigenschaften anderer Gläser und Glaskeramiken eignen.

Diese Aufgabe wird erfindungsgemäß gelöst durch ein Glas oder eine Glaskeramik mit Gehalt an Europium, die die folgenden Komponenten enthalten:

| | |
|---|---|
| SiO₂ | 30,0 bis 75,0 |
| Al₂O₃ | 10, 0 bis 45,0 |
| Europium, berechnet als Eu₂O₃ | 0,05 bis 5,0. |

Es hat sich überraschend gezeigt, dass das erfindungsgemäße Glas und die erfindungsgemäße Glaskeramik gegenüber dem Stand der Technik verbesserte Fluoreszenzeigenschaften bei Anregungswellenlängen im Bereich von 250 bis 430 nm und insbesondere auch bei Anregungswellenlängen im Bereich von 360 bis 430 nm zeigen und diese Fluoreszenzeigenschaften zudem weitgehend stabil gegenüber Wärmebehandlungen und oxidierenden Bedingungen sind.

Ohne Beschränkung auf eine bestimmte Theorie wird angenommen, dass es in den erfindungsgemäßen Gläsern und Glaskeramiken zur Ausbildung eines Gleichgewichts zwischen Eu²⁺ und Eu³⁺-Ionen kommt. Die Eu²⁺-Ionen zeigen aufgrund von 5d→4f-Übergängen eine Fluoreszenz bei Anregungswellenlängen im gesamten Bereich von 250 bis 430 nm, die in besonderer Weise geeignet ist, die Fluoreszenzeigenschaften des natürlichen Zahnmaterials insbesondere bei Anregung im UV-Bereich zu imitieren.

Erfindungsgemäß ist es bevorzugt, dass das Glas und die Glaskeramik 32,0 bis 72,0, insbesondere 35,0 bis 65,0 und bevorzugt 38,0 bis 50,0 Gew.-% SiO₂ enthalten.

Weiter ist es bevorzugt, dass das Glas und die Glaskeramik 15,0 bis 40,0, insbesondere 20,0 bis 40,0, bevorzugt 25,0 bis 40,0 und besonders bevorzugt 30,0 bis 40,0 Gew.-% Al₂O₃ enthalten.

Vorzugsweise enthalten das Glas und die Glaskeramik 0,1 bis 4,0, insbesondere 0,3 bis 3,0, bevorzugt 0,5 bis 2,0, weiter bevorzugt 0,6 bis 1,0 und besonders bevorzugt 0,65 bis 0,85 Gew.-% Europium, berechnet als Eu₂O₃.

Es ist weiter bevorzugt, dass das Glas und die Glaskeramik 8,0 bis 30,0 Gew.-%, insbesondere 12,0 bis 29,0, bevorzugt 15,0 bis 28,0 und besonders bevorzugt 20,0 bis 27,0 Gew.-% Me^{II}O enthalten, wobei Me^{II}O ausgewählt ist aus MgO, CaO, SrO und/oder ZnO. In einer bevorzugten Ausführungsform enthalten das Glas und die Glaskeramik 8,0 bis 30,0, insbesondere 12,0 bis 29,0, bevorzugt 15,0 bis 28,0 und besonders bevorzugt 20,0 bis 27,0 Gew.-% CaO und/oder SrO.

Besonders bevorzugt sind Gläser und Glaskeramiken, die mindestens eine und bevorzugt alle der folgenden Komponenten in den angegebenen Mengen enthalten:

| Komponente | Gew.-% |
|---|---|
| MgO | 0 bis 13,0, insbesondere 3,5 bis 13,0 |
| CaO | 0 bis 22,0, insbesondere 5,0 bis 22,0 |
| SrO | 0 bis 28,0, insbesondere 9,0 bis 28,0 |
| ZnO | 0 bis 5,0, insbesondere 4,0 bis 5,0. |

Weiterhin ist es bevorzugt, dass das Glas und die Glaskeramik 0 bis 10,0, insbesondere 0 bis 5,0 und bevorzugt 0 bis 1,0 Gew.-% BaO enthalten und am meisten bevorzugt im Wesentlichen frei von BaO sind.

In einer bevorzugten Ausführungsform enthalten das Glas und die Glaskeramik 0 bis 2,0, insbesondere 0,1 bis 1,2 und bevorzugt 0,3 bis 0,7 Gew.-% Zinn, berechnet als SnO.

Bevorzugt enthalten das Glas und die Glaskeramik ferner 0 bis 5,0, vorzugsweise 0,5 bis 4,0 und bevorzugt 1,0 bis 3,0 Gew.-% Cer, berechnet als CeO₂.

Das Glas und die Glaskeramik können ferner Alkalimetalloxid Me^{I}₂O in einer Menge von 0 bis 15,0, insbesondere 0 bis 10,0, bevorzugt 0 bis 5,0, besonders bevorzugt 0 bis 1,0 und am meisten bevorzugt 0 bis 0,5 Gew.-% enthalten, wobei dieses Me^{I}₂O insbesondere ausgewählt ist aus Li₂O, Na₂O, K₂O, Rb₂O und Cs₂O und vorzugsweise ausgewählt ist aus Li₂O, Na₂O und K₂O. Besonders bevorzugt enthalten das Glas und die Glaskeramik mindestens eines und insbesondere alle der folgenden Alkalimetalloxide Me^{I}₂O in den angegebenen Mengen:

| Komponente | Gew.-% |
|---|---|
| Li₂O | 0 bis 5,0 |
| Na₂O | 0 bis 10,0 |
| K₂O | 0 bis 14,0 |
| Rb₂O | 0 bis 7,0 |
| Cs₂O | 0 bis 13,0. |

Das Glas und die Glaskeramik können ferner 0 bis 10,0, insbesondere 0 bis 4,0 und bevorzugt 0 bis 2,5 Gew.-% weiteres Oxid dreiwertiger Elemente Me^{III}₂O₃ enthalten. Der Begriff "weiteres Oxid dreiwertiger Elemente Me^{III}₂O₃" bezeichnet dreiwertige Oxide mit Ausnahme von B₂O₃, Al₂O₃, Eu₂O₃ und Ce₂O₃, wobei dieses Me^{III}₂O₃ insbesondere ausgewählt ist aus Y₂O₃, La₂O₃, Ga₂O₃ und/oder In₂O₃. Besonders bevorzugt enthalten das Glas und die Glaskeramik mindestens eines und insbesondere alle der folgenden weiteren Oxide dreiwertiger Elemente Me^{III}₂O₃ in den angegebenen Mengen:

| Komponente | Gew.-% |
|---|---|
| Y₂O₃ | 0 bis 3,0 |
| La₂O₃ | 0 bis 2,0 |
| Ga₂O₃ | 0 bis 2,0 |
| In₂O₃ | 0 bis 1,0. |

Weiterhin ist es bevorzugt, dass das Glas und die Glaskeramik 0 bis 3,0, insbesondere 0 bis 2,0 und bevorzugt 0 bis 1,0 Gew.-% B₂O₃ enthalten und am meisten bevorzugt im Wesentlichen frei von B₂O₃ sind.

Ferner können das Glas und die Glaskeramik weiteres Oxid vierwertiger Elemente Me^{IV}O₂ in einer Menge 0 bis 15,0, insbesondere 0 bis 4,0 und besonders bevorzugt 0 bis 2,5 Gew.-% enthalten. Der Begriff "weiteres Oxid vierwertiger Elemente Me^{IV}O₂" bezeichnet vierwertige Oxide mit Ausnahme von SiO₂, SnO₂, CeO₂ und TiO₂, wobei dieses Me^{IV}O₂ insbesondere ausgewählt ist aus ZrO₂ und/oder GeO₂. Besonders bevorzugt enthalten das Glas und die Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 bis 15,0 |
| GeO₂ | 0 bis 5,0. |

Weiterhin ist es bevorzugt, dass das Glas und die Glaskeramik 0 bis 5,0, insbesondere 0 bis 2,5 und bevorzugt 0 bis 1,0 Gew.-% TiO₂ enthält und am meisten bevorzugt im Wesentlichen frei von TiO₂ ist.

Außerdem können das Glas und die Glaskeramik Oxid fünfwertiger Elemente Me^{V}₂O₅ in einer Menge 0 bis 6,0 und insbesondere 0 bis 5,0 Gew.-% enthalten, wobei dieses Me^{V}₂O₅ insbesondere ausgewählt ist aus P₂O₅, V₂O₅, Ta₂O₅ und/oder Nb₂O₅. Besonders bevorzugt enthalten das Glas und die Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| P₂O₅ | 0 bis 6,0 |
| V₂O₅ | 0 bis 6,0 |
| Ta₂O₅ | 0 bis 5,0 |
| Nb₂O₅ | 0 bis 5,0. |

Das Glas und die Glaskeramik können zudem 0 bis 6,0 Gew.-% Oxid sechswertiger Elemente Me^{VI}O₃ enthalten, wobei dieses Me^{VI}O₃ insbesondere ausgewählt ist aus WO₃ und/oder MoO₃. Besonders bevorzugt enthalten das Glas und die Glaskeramik mindestens eines und insbesondere alle der folgenden Oxide Me^{VI}O₃ in den angegebenen Mengen:

| Komponente | Gew.-% |
|---|---|
| WO₃ | 0 bis 6, 0 |
| MoO₃ | 0 bis 5,0. |

Das Glas und die Glaskeramik können ferner Oxide von weiteren f-Elementen, wie z.B. Oxide von Pr, Nd, Gd, Tb, Dy, Er und Yb und insbesondere Oxide von Tb und/oder Dy enthalten.

Weiterhin können das Glas und die Glaskeramik 0 bis 5,0 und insbesondere 0 bis 1,0 Gew.-% Fluor enthalten.

Besonders bevorzugt sind ein Glas und eine Glaskeramik, die mindestens eine und bevorzugt alle der folgenden Komponenten in den angegebenen Mengen enthalten:

| Komponente | Gew.-% |
|---|---|
| SiO₂ | 32,0 bis 72,0 |
| Al₂O₃ | 15,0 bis 40,0 |
| Europium, berechnet als Eu₂O₃ | 0,1 bis 4,0 |
| Cer, berechnet als CeO₂ | 0 bis 5,0 |
| Zinn, berechnet als SnO | 0 bis 2,0 |
| Me^{I}₂O | 0 bis 15,0 |
| Me^{II}O | 0 bis 30, 0 |
| Me^{III}₂O₃ | 0 bis 10,0 |
| Me^{IV}O₂ | 0 bis 15,0 |
| Me^{V}₂O₅ | 0 bis 6,0 |
| Me^{VI}O₃ | 0 bis 6,0 |
| Fluor | 0 bis 5,0, |

wobei Me^{I}₂O, Me^{II}O, Me^{III}₂O₃, Me^{IV}O₂, Me^{V}₂O₅ und Me^{VI}O₃ insbesondere die oben angegebenen Bedeutungen haben.

In einer weiteren besonders bevorzugten Ausführungsform enthalten das Glas und die Glaskeramik mindestens eine und bevorzugt alle der folgenden Komponenten in den angegebenen Mengen:

| Komponente | Gew.-% |
|---|---|
| SiO₂ | 32,0 bis 72,0 |
| Al₂O₃ | 15,0 bis 40,0 |
| Europium, berechnet als Eu₂O₃ | 0,1 bis 4,0 |
| Cer, berechnet als CeO₂ | 0 bis 5,0 |
| Zinn, berechnet als SnO | 0 bis 2,0 |
| Li₂O | 0 bis 5,0 |
| Na₂O | 0 bis 10,0 |
| K₂O | 0 bis 14,0 |
| Rb₂O | 0 bis 7,0 |
| Cs₂O | 0 bis 13,0 |
| MgO | 0 bis 13,0 |
| CaO | 0 bis 22,0 |
| SrO | 0 bis 28,0 |
| ZnO | 0 bis 5,0 |
| BaO | 0 bis 10,0 |
| B₂O₃ | 0 bis 3,0 |
| Y₂O₃ | 0 bis 3,0 |
| La₂O₃ | 0 bis 2,0 |
| Ga₂O₃ | 0 bis 2,0 |
| In₂O₃ | 0 bis 1,0 |
| ZrO₂ | 0 bis 15,0 |
| GeO₂ | 0 bis 5,0 |
| TiO₂ | 0 bis 5,0 |
| P₂O₅ | 0 bis 6,0 |
| V₂O₅ | 0 bis 6,0 |
| Ta₂O₅ | 0 bis 5,0 |
| Nb₂O₅ | 0 bis 5,0 |
| WO₃ | 0 bis 6,0 |
| MoO₃ | 0 bis 5,0 |
| Dy₂O₃ | 0 bis 3,0 |
| Tb₄O₇ | 0 bis 2,0 |
| Fluor | 0 bis 1,0. |

Die erfindungsgemäße Glaskeramik enthält vorzugsweise mindestens ein Alumosilikat als Kristallphase und insbesondere als Hauptkristallphase. In einer weiter bevorzugten Ausführungsform enthält die erfindungsgemäße Glaskeramik ein Calciumalumosilikat oder Strontiumalumosilikat oder eine Mischung von diesen, bevorzugt ein Calciumalumosilikat oder Strontiumalumosilikat, als Kristallphase und insbesondere als Hauptkristallphase. Ohne Beschränkung auf eine bestimmte Theorie wird angenommen, dass es in den erfindungsgemäßen Glaskeramiken zu einem Einbau von Eu²⁺-Ionen in das Kristallgitter der vorstehend genannten Kristallphasen kommt und dadurch deren Fluoreszenzeigenschaften weiter verbessert und stabilisiert werden.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Es ist weiter bevorzugt, dass die erfindungsgemäße Glaskeramik mindestens 5 Gew.-%, insbesondere mindestens 10 Gew.-% und bevorzugt mindestens 20 Gew.-% Alumosilikat als Kristallphase, insbesondere in Form von Calciumalumosilikat, Strontiumalumosilikat oder Mischungen von diesen, enthält.

Die Art und die Menge der gebildeten Kristallphasen können insbesondere durch die Zusammensetzung des zugrundeliegenden Glases sowie die Wärmebehandlung gesteuert werden, die zur Herstellung der Glaskeramik aus dem Glas angewendet wird. Die Beispiele veranschaulichen dies anhand der Variation der Zusammensetzung und der angewendeten Wärmebehandlung.

Die Erfindung betrifft ebenfalls Vorstufen mit entsprechender Zusammensetzung, aus denen die erfindungsgemäße Glaskeramik durch Wärmebehandlung hergestellt werden kann. Diese Vorstufen sind ein entsprechend zusammengesetztes Glas (auch als Ausgangsglas bezeichnet) und ein entsprechend zusammengesetztes Glas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft ebenfalls ein erfindungsgemäßes Glas, das Keime für die Kristallisation von einem Alumosilikat und insbesondere von Calciumalumosilikat und/oder Strontiumalumosilikat enthält. Durch Wärmebehandlung des erfindungsgemäßen Glases kann zunächst das erfindungsgemäße Glas mit Keimen erzeugt werden, welches seinerseits durch weitere Wärmebehandlung in die erfindungsgemäße Glaskeramik, insbesondere mit einem Alumosilikat als Kristallphase und vorzugsweise als Hauptkristallphase, umgewandelt werden kann.

Die Herstellung des erfindungsgemäßen Glases erfolgt insbesondere in der Weise, dass eine Mischung von geeigneten Ausgangsmaterialien, wie z.B. Carbonaten, Oxiden, Phosphaten und Fluoriden, bei Temperaturen von insbesondere 1500 bis 1800°C für 0,5 bis 10 h erschmolzen und die erhaltene Glasschmelze in Wasser gegeben wird, um ein Granulat herzustellen. Dieses Granulat kann dann nach Mahlen zu einem Rohling, einem sogenannten Pulverpressling, gepresst oder zu einem Pulver verarbeitet werden.

Die Erfindung ist daher ebenfalls auf ein Verfahren zur Herstellung der erfindungsgemäßen Glaskeramik gerichtet, bei dem das Glas, insbesondere das Glas mit Keimen, mindestens einer Wärmebehandlung bei einer Temperatur von 1000 bis 1500°C, vorzugsweise 1050 bis 1450°C, für eine Dauer von insbesondere 10 bis 720 min und vorzugsweise 30 bis 120 min unterworfen wird.

Somit betrifft die Erfindung in einer bevorzugten Ausführungsform ein Verfahren zur Herstellung der erfindungsgemäßen Glaskeramik, bei dem
(a) Pulver des erfindungsgemäßen Glases, gegebenenfalls nach Zugabe weiterer Komponenten, wie z.B. anderen Gläsern, Glaskeramiken und/oder Presshilfsmittel, zu einem Pulverpressling gepresst wird, und
(b) der Pulverpressling einer Wärmebehandlung bei einer Temperatur von 1000 bis 1500°C, vorzugsweise 1050 bis 1450°C, für eine Dauer von insbesondere 10 bis 720 min, vorzugsweise 30 bis 120 min, unterworfen wird.

Gegebenenfalls kann vor der Wärmebehandlung in Schritt (b) eine Keimbildung vorgenommen werden.

Gegebenenfalls kann die Schmelze des Ausgangsglases mit mindestens einem reduzierenden Mittel umgesetzt werden. Als reduzierende Mittel kommen grundsätzlich alle Mittel in Frage, die unter den Bedingungen des Verfahrens zur Reduktion von Eu³⁺-Ionen zu Eu²⁺-Ionen in der Lage sind. Dabei sind solche reduzierenden Mittel bevorzugt, die nach der Reduktion rückstandsfrei aus der Glasschmelze entfernt werden können.

Insbesondere sind gasförmige reduzierende Mittel sowie reduzierende Mittel bevorzugt, die unter den Bedingungen des erfindungsgemäßen Verfahrens nach der Reduktion aus der Glasschmelze ausgebrannt werden. Beispiele für gasförmige reduzierende Mittel sind Gase, die Wasserstoff und bevorzugt Mischungen von Wasserstoff und Stickstoff enthalten. Beispiele für reduzierende Mittel sind ferner Stoffe, die mindestens ein oxidierbares Kohlenstoffatom enthalten, insbesondere Kohlenstoff, beispielsweise Graphit, organische Salze, Kohlenhydrate und Getreidemehle.

Gemäß einer bevorzugten Ausführungsform wird die Schmelze des Ausgangsglases aus einer glasbildenden Zusammensetzung gebildet, die mindestens ein reduzierendes Mittel enthält. Dabei ist als das mindestens eine reduzierende Mittel eine Verbindung bevorzugt, die mindestens ein oxidierbares Kohlenstoffatom enthält und vorzugsweise aus der Gruppe bestehend aus organischen Salzen, Kohlenhydraten und Getreidemehlen ausgewählt ist. Beispiele für besonders geeignete organische Salze sind Acetate.

In einer besonders bevorzugten Ausführungsform wird als reduzierendes Mittel ein Europiumacetat, insbesondere Europium(III)acetat-hydrat verwendet.

Gemäß einer weiteren bevorzugten Ausführungsform ist das mindestens eine reduzierende Mittel ein reduzierendes Gas, wobei das Gas vorzugsweise Wasserstoff enthält und bevorzugt Wasserstoff und Stickstoff enthält. Besonders geeignet sind Mischungen von Wasserstoff und Stickstoff, die etwa 5 Vol.-% Wasserstoff enthalten und auch als Formiergas bezeichnet werden. Dabei kann das Ausmaß der Reduktion über die Menge des zugeführten Gases und insbesondere über die Flussrate und Dauer der Zuführung des Gases gesteuert werden. Vorzugsweise beträgt die Menge der wirksamen Komponente des reduzierenden Gases, vorzugsweise Wasserstoff, 0,05 bis 5 l/min, insbesondere 0,1 bis 1 l/min und bevorzugt 0,2 bis 0,5 l/min, für eine Dauer von 10 bis 180 min, insbesondere 20 bis 120 min und bevorzugt 30 bis 90 min.

Die Erfindung betrifft ferner ein erfindungsgemäßes Glas und eine erfindungsgemäße Glaskeramik, die eine weißlich-blaue Fluoreszenzfarbe im CIE-Farbraum aufweisen.

Die erfindungsgemäßen Gläser und Glaskeramiken mit Gehalt an Europium eignen sich insbesondere als Mischungskomponenten zur Einstellung der Fluoreszenzeigenschaften anderer Gläser und Glaskeramiken. Ein Glas oder eine Glaskeramik, die das erfindungsgemäße Glas oder die erfindungsgemäße Glaskeramik mit Gehalt an Europium enthalten, stellen daher einen weiteren Gegenstand der Erfindung dar. Dabei sind ein Glas und eine Glaskeramik besonders bevorzugt, die das erfindungsgemäße Glas oder die erfindungsgemäße Glaskeramik mit Gehalt an Europium in einer Menge von 0,1 bis 50 Gew.-%, insbesondere 0,2 bis 40 Gew.-%, bevorzugt 0,5 bis 30 Gew.-%, besonders bevorzugt 1 bis 20 Gew.-% und ganz besonders bevorzugt 5 bis 10 Gew.-% enthalten.

Das erfindungsgemäße Glas oder die erfindungsgemäße Glaskeramik mit Gehalt an Europium können insbesondere als Komponente eines anorganisch-anorganischen Komposits oder in Kombination mit einer Vielzahl von anderen Gläsern und/oder Glaskeramiken verwendet werden, wobei die Komposite oder Kombinationen insbesondere als Dentalmaterialien eingesetzt werden können. Besonders bevorzugt können die Komposite oder Kombinationen in Form von Sinterrohlingen vorliegen. Beispiele anderer Gläser und Glaskeramiken zur Herstellung von anorganisch-anorganischen Kompositen und von Kombinationen sind in DE 43 14 817 A1, DE 44 23 793 Cl, DE 44 23 794 C1, DE 44 28 839 A1, DE 196 47 739 A1, DE 197 25 552 A1, DE 100 31 431 A1, EP 0 827 941 A1, EP 0 916 625 A1, WO 00/34196 A2, EP 1 505 041 A1, EP 1 688 398 A1, EP 2 287 122 A1, EP 2 377 831 A1, EP 2 407 439 A1, WO 2013/053863 A2, WO 2013/053864 A2, WO 2013/053865 A2, WO 2013/053866 A2, WO 2013/053867 A2, WO 2013/053868 A2, WO 2013/164256 A1, WO 2014/170168 A1, WO 2014/170170 A2, WO 2015/067643 A1, WO 2015/155038 A1, WO 2015/173394 A1, WO 2016/120146 A1, WO 2017/032745 A1, WO 2017/055010 A1 offenbart. Diese Gläser und Glaskeramiken gehören zur Silikat-, Borat-, Phosphat- oder Alumosilikat-Gruppe. Bevorzugte Gläser und Glaskeramiken sind vom SiO₂-Al₂O₃-K₂O-Typ (mit kubischen oder tetragonalen Leucit-Kristallen), SiO₂-B₂O₃-Na₂O-Typ, Alkali-Silikat-Typ, Alkali-Zink-Silikat-Typ, Silico-Phosphat-Typ und/oder SiO₂-ZrO₂-Typ. Besonders bevorzugt sind Lithiumsilikat-Glaskeramiken und insbesondere Glaskeramiken, die Lithiummetasilikat oder Lithiumdisilikat als Hauptkristallphase und gegebenenfalls weitere Kristallphasen wie Apatit, Diopsid, Quarz und/oder Wollastonit enthalten, sowie Glaskeramiken, die SiO₂, insbesondere in Form von Tiefquarz, als Hauptkristallphase enthalten. Durch Vermischen derartiger Gläser oder Glaskeramiken mit den erfindungsgemäßen Gläsern und/oder Glaskeramiken mit Gehalt an Europium können insbesondere die Fluoreszenzeigenschaften in gewünschter Weise eingestellt werden.

Die erfindungsgemäßen Glaskeramiken und die erfindungsgemäßen Gläser, insbesondere in Form von Kompositen und Kombinationen, liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden, z.B. zu dentalen Restaurationen. Sie können aber auch in Form von dentalen Restaurationen, wie Inlays, Onlays, Kronen, Teilkronen, Brücken, Veneers, Schalen oder Abutments, vorliegen.

Aus den erfindungsgemäßen Glaskeramiken und den erfindungsgemäßen Gläsern, insbesondere in Form von Kompositen und Kombinationen, können dentale Restaurationen, wie Inlays, Onlays, Kronen, Teilkronen, Brücken, Veneers, Schalen oder Abutments, hergestellt werden. Die Erfindung betrifft daher deren Verwendung als Dentalmaterial und insbesondere deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1150°C und insbesondere 700 bis 1000°C erfolgt. Weiter ist es bevorzugt, das Verpressen bei einem Druck von 10 bis 30 bar durchzuführen. Beim Verpressen wird durch viskoses Fließen des eingesetzten Materials die gewünschte Formänderung erreicht. Es können für das Verpressen das erfindungsgemäße Glas und das erfindungsgemäße Glas mit Keimen sowie bevorzugt die erfindungsgemäße Glaskeramik verwendet werden. Dabei können die erfindungsgemäßen Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Glas, das erfindungsgemäße Glas mit Keimen sowie die erfindungsgemäße Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Glaskeramik verwendet. Die erfindungsgemäße Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich sind. Nach der maschinellen Bearbeitung eines solchen teilkristallisierten Materials wird dieses regelmäßig einer weiteren Wärmebehandlung unterzogen, um eine weitere Kristallisation hervorzurufen.

Die erfindungsgemäßen Glaskeramiken und die erfindungsgemäßen Gläser, insbesondere in Form von Kompositen und Kombinationen, eignen sich allerdings auch als Beschichtungsmaterial von z.B. Keramiken, Glaskeramiken und Metallen. Die Erfindung ist daher ebenfalls auf die Verwendung der erfindungsgemäßen Gläser oder der erfindungsgemäßen Glaskeramiken zur Beschichtung von insbesondere Keramiken, Glaskeramiken und Metallen gerichtet.

Die Erfindung betrifft auch ein Verfahren zur Beschichtung von Keramiken, Glaskeramiken und Metallen, bei dem erfindungsgemäße Glaskeramik oder erfindungsgemäßes Glas, insbesondere in Form von Kompositen und Kombinationen, auf die Keramik, die Glaskeramik oder das Metall aufgebracht und einer Temperatur von mindestens 600°C ausgesetzt wird.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik, Glaskeramik oder Metall, aufgebracht und anschließend gesintert. Bei dem bevorzugten Aufpressen wird erfindungsgemäße Glaskeramik oder erfindungsgemäßes Glas, z.B. in Form von Pulverpresslingen oder monolithischen Rohlingen, bei einer erhöhten Temperatur, von z.B. 700 bis 1150°C und insbesondere 700 bis 1000°C, und unter Anwendung von Druck, z.B. 10 bis 30 bar, aufgepresst. Hierzu können insbesondere die in der EP 231 773 beschriebenen Verfahren und der dort offenbarte Pressofen eingesetzt werden. Geeignete kommerzielle Öfen sind die Öfen vom Typ Programat von Ivoclar Vivadent AG, Liechtenstein.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser, insbesondere in Form von Kompositen und Kombinationen, als Dentalmaterial und insbesondere zur Herstellung dentaler Restaurationen oder als Beschichtungsmaterial für dentale Restaurationen, wie Kronen, Brücken und Abutments.

Die Erfindung betrifft mithin auch ein Verfahren zur Herstellung einer dentalen Restauration, insbesondere Inlay, Onlay, Krone, Teilkrone, Brücke, Veneer, Schale oder Abutment, bei dem der erfindungsgemäßen Glaskeramik oder dem erfindungsgemäßen Glas, insbesondere in Form von Kompositen und Kombinationen, durch Verpressen, Sintern oder maschinelle Bearbeitung, insbesondere im Rahmen eines CAD/CAM-Verfahrens, die Form der gewünschten dentalen Restauration gegeben wird.

Die Erfindung wird im Folgenden anhand von sie nicht beschränkenden Beispielen näher erläutert.

### Beispiele

Es wurden insgesamt 21 erfindungsgemäße Gläser mit den in der Tabelle I angegebenen Zusammensetzungen hergestellt, wobei sich die Oxidationsstufen der angegebenen Oxide auf die Oxidationsstufen der eingesetzten Rohstoffe beziehen. Die Gläser wurden gemäß Tabelle II zu Glaskeramiken kristallisiert. Dabei bedeuten

| | |
|---|---|
| T_{g} | Glasübergangstemperatur, bestimmt mittels DSC |
| T_{S} und t_{S} | angewendete Temperatur und Zeit zum Erschmelzen |
| T_{Sinter} und t_{Sinter} | angewendete Temperatur und Zeit für Sintern. |

Zudem wurden die Gläser oder Glaskeramiken gemäß Tabelle III mit weiteren Gläsern gemischt und zu glaskeramischen Körpern gesintert.

### A) Herstellung erfindungsgemäßer Gläser

In den Beispielen wurden zunächst Ausgangsgläser mit den in der Tabelle I angegebenen Zusammensetzungen im 100 bis 200 g Maßstab aus üblichen Rohstoffen bei der Temperatur T_{S} für eine Dauer t_{S} erschmolzen. Durch Eingießen der erschmolzenen Ausgangsgläser in Wasser wurden Glasfritten hergestellt. Die Fluoreszenzeigenschaften der so erhaltenen Glasfritten bei Anregungswellenlängen von 254, 366, 395 bzw. 430 nm wurden visuell mittels einer UV-Lampe bestimmt und sind in Tabelle II wiedergegeben. Danach zeigten insbesondere bei Anregungswellenlängen von 366, 395 und 430 nm alle in den Beispielen erhaltenen Gläser eine Fluoreszenz.

Für die Weiterverarbeitung der Glasfritten wurden die nachstehend angegebenen drei Verfahrensvarianten B) bis D) benutzt:

### B) Herstellung erfindungsgemäßer Glaskeramiken

In den Beispielen 1 bis 13 wurden die erhaltenen Glasfritten in einer Zirkonoxidmühle auf eine Korngröße von < 45 µm aufgemahlen. Ca. 4 g dieser Pulver wurden anschließend zu zylinderförmigen Rohlingen verpresst und gemäß Tabelle II bei einer Temperatur T_{Sinter} und einer Haltezeit von t_{Sinter} zu dichten glaskeramischen Körpern gesintert, wobei dies für Sintertemperaturen bis 1200°C in einem Sinterofen vom Typ Programat® (Ivoclar Vivadent AG) im Vakuum und für Sintertemperaturen oberhalb von 1200°C in einem Sinterofen vom Typ LHT 02/16 (Nabertherm) in normaler Atmosphäre erfolgte. Die Fluoreszenzeigenschaften der so erhaltenen Glaskeramiken bei Anregungswellenlängen von 254, 366, 395 bzw. 430 nm wurden visuell mittels einer UV-Lampe bestimmt und sind in Tabelle II wiedergegeben. Danach zeigten insbesondere bei Anregungswellenlängen von 366, 395 und 430 nm die erhaltenen Glaskeramiken eine Fluoreszenz.

### C) Erfindungsgemäße Gläser als Mischungskomponenten

Die in den Beispielen 14 und 21 erhaltenen Glasfritten wurden zerkleinert und auf eine Korngröße von < 25 µm gesiebt. Die erhaltenen Glaspulver wurden gemäß Tabelle III pulverisierten Lithiumsilikatgläsern zugemischt. Jeweils ca. 4 g dieser Mischungen wurden anschließend zu zylinderförmigen oder scheibenförmigen Rohlingen verpresst und in einem Sinterofen (Programat® von Ivoclar Vivadent AG) zu dichten glaskeramischen Körpern gesintert. Die Fluoreszenzeigenschaften der so erhaltenen glaskeramischen Körper bei Anregungswellenlängen von 254, 366, 395 bzw. 430 nm wurden visuell mittels einer UV-Lampe bestimmt und sind in Tabelle III wiedergegeben. Danach zeigten insbesondere bei Anregungswellenlängen von 366, 395 und 430 nm alle erhaltenen glaskeramischen Körper eine Fluoreszenz.

### D) Erfindungsgemäße Glaskeramiken als Mischungskomponenten

Die in den Beispielen 1, 2, 4 und 9 erhaltenen Glaskeramiken und die in Beispiel 5 nach Sinterung bei 1110°C erhaltene Glaskeramik wurden zerkleinert und auf eine Korngröße von < 25 µm gesiebt. Die erhaltenen Glaskeramikpulver wurden gemäß Tabelle III pulverisierten Lithiumsilikatgläsern zugemischt. Jeweils ca. 4 g dieser Mischungen wurden anschließend zu zylinderförmigen oder scheibenförmigen Rohlingen oder zu Pressstümpfen verpresst und in einem Sinterofen (Programat® von Ivoclar Vivadent AG) zu dichten glaskeramischen Körpern gesintert oder heißgepresst. Die Fluoreszenzeigenschaften der so erhaltenen glaskeramischen Körper bei Anregungswellenlängen von 254, 366, 395 bzw. 430 nm wurden visuell mittels einer UV-Lampe bestimmt und sind in Tabelle III wiedergegeben. Danach zeigten insbesondere bei Anregungswellenlängen von 366 nm alle erhaltenen glaskeramischen Körper eine Fluoreszenz. Als Kristallphasen wurden in CaO-haltigen Zusammensetzungen Calciumalumosilikate wie Anorthit und in SrO-haltigen Zusammensetzungen Strontiumalumosilikate wie Slawsonit erhalten.

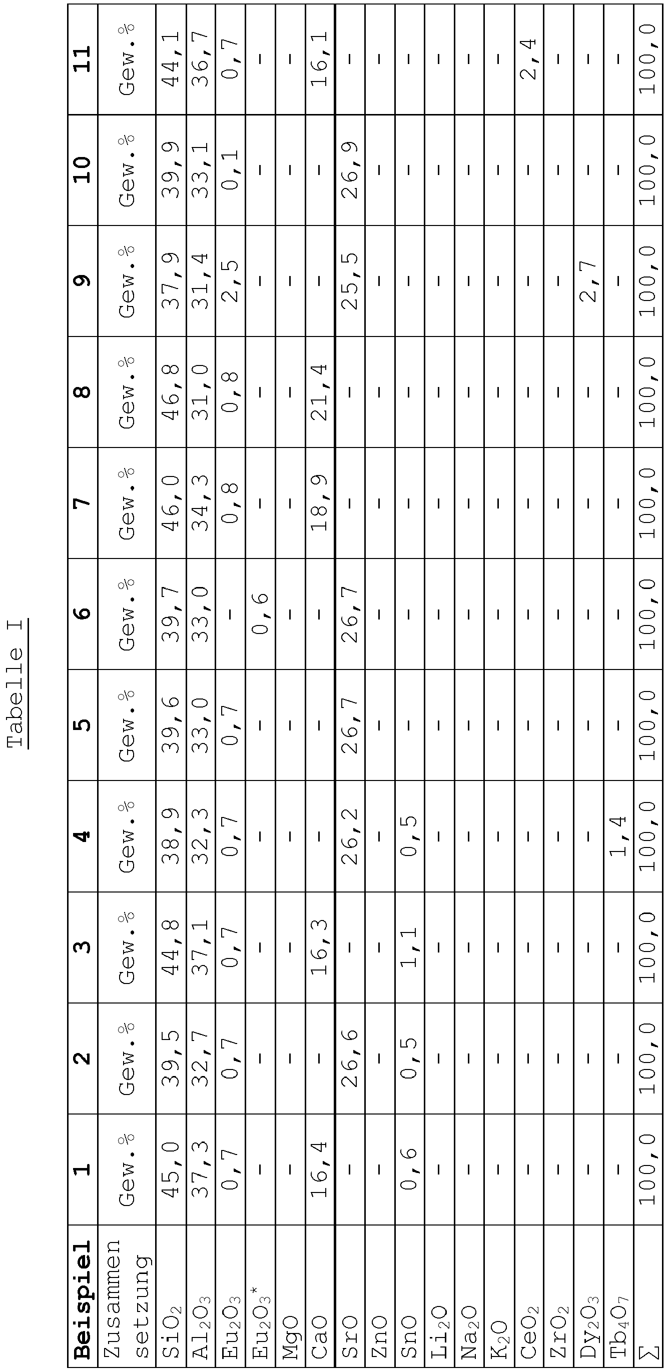

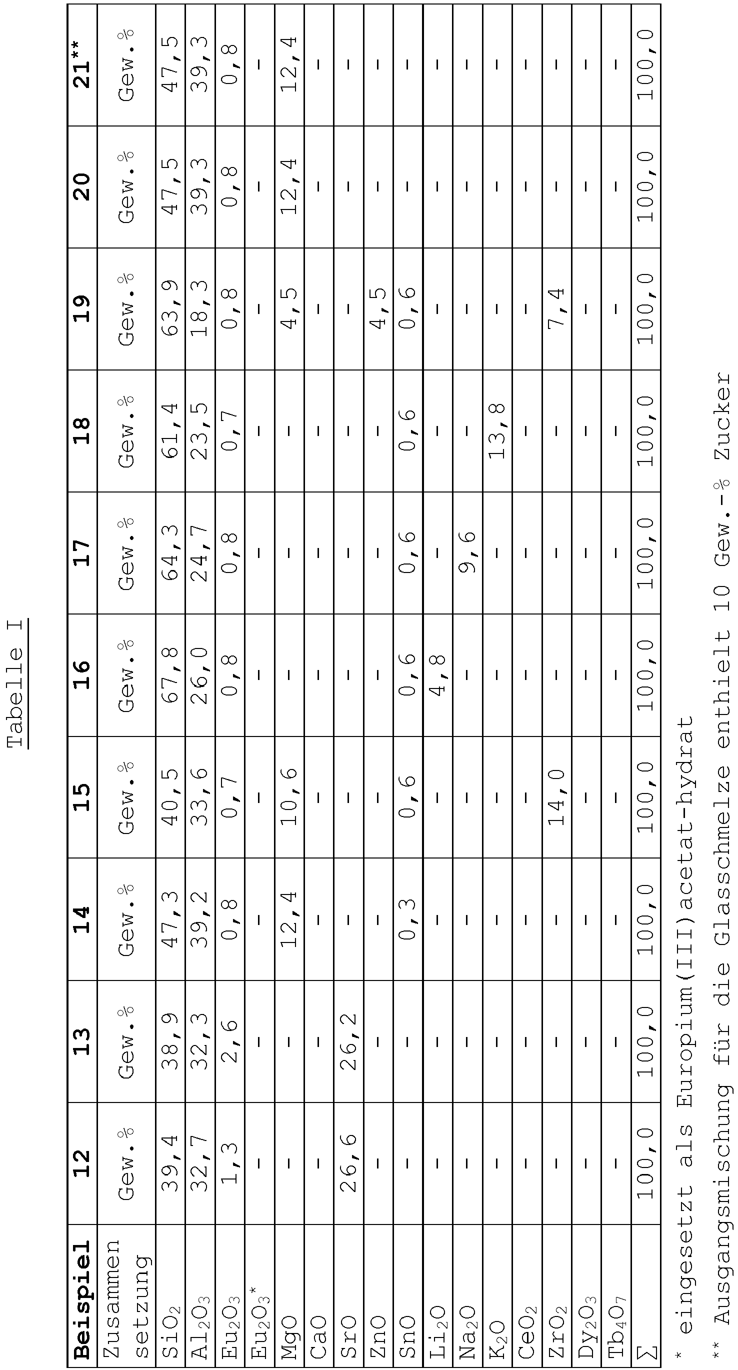

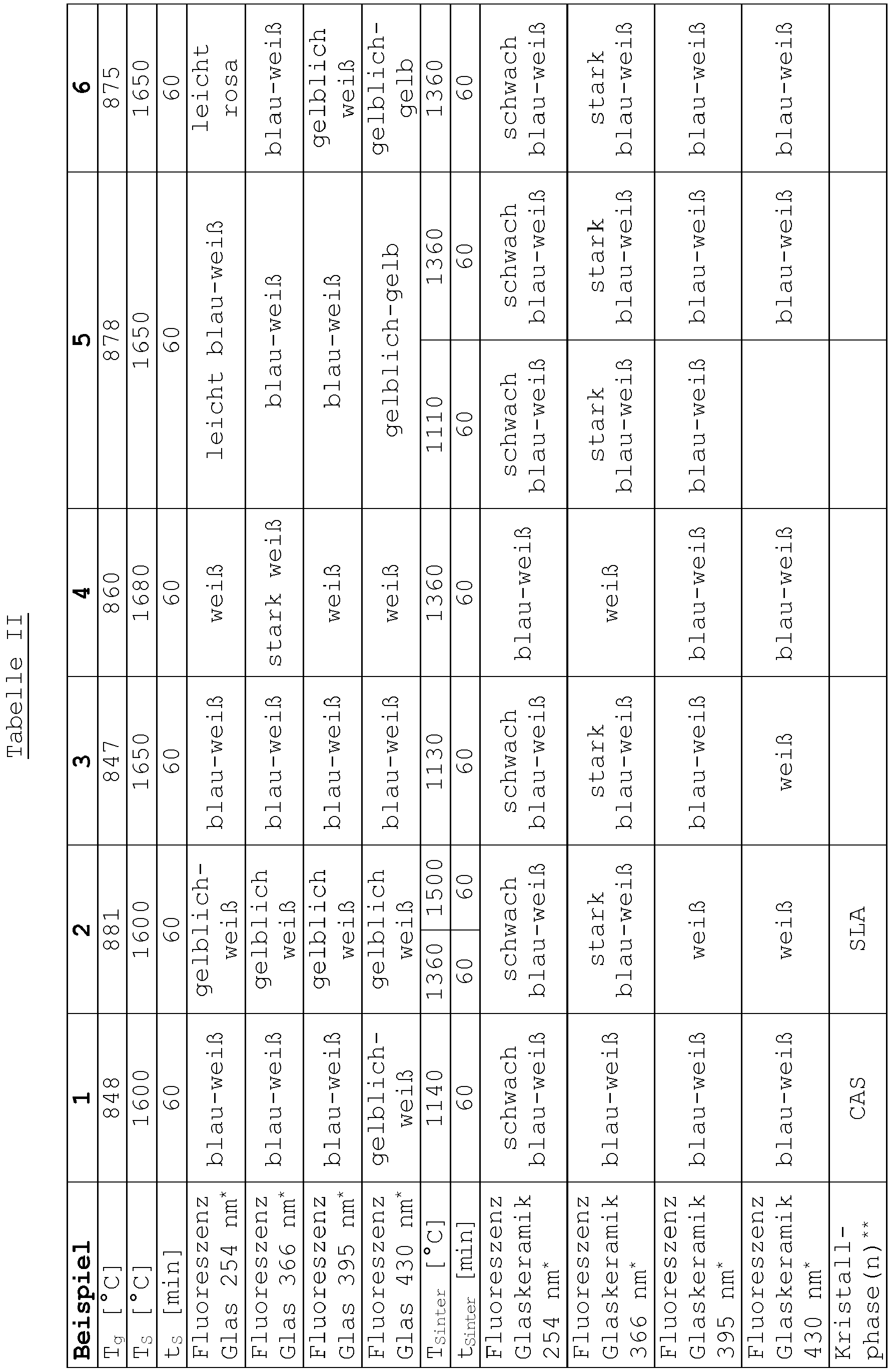

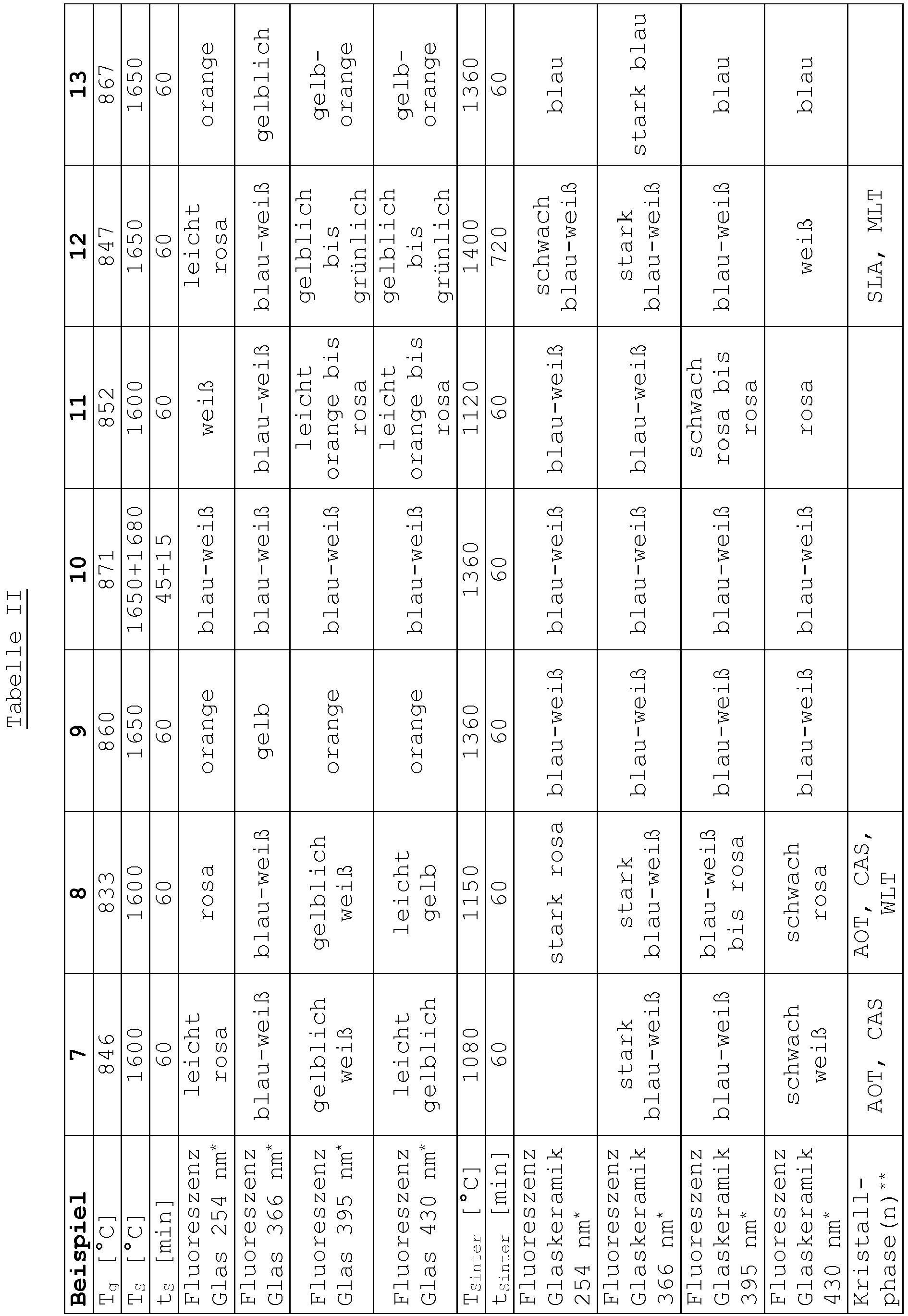

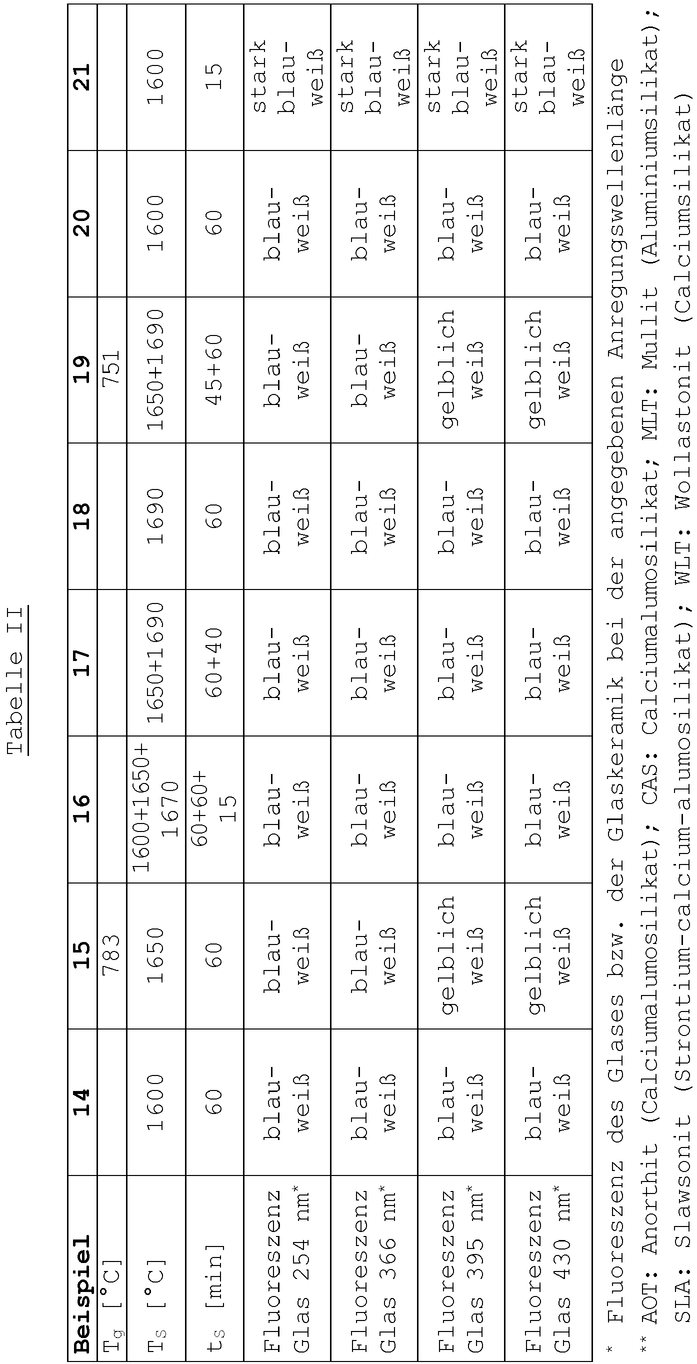

**Tabelle III**

| Komponente A | %A** | Komponente B | %B** | Wärmebehandlung | Fluoreszenz 254 nm* | Fluoreszenz 366 nm* | Fluoreszenz 395 nm* | Fluoreszenz 430 nm* |
|---|---|---|---|---|---|---|---|---|
| Glas nach Beispiel 14 | 10 | Glas gemäß Beispiel 10 der WO2016/120146A1 | 90 | Sintern 790°C/10 min (Zylinder) | | blau-weiß | schwach weiß | schwach weiß |
| Glas nach Beispiel 14 | 10 | Glas gemäß Beispiel 31 der WO2017/055010A1 | 90 | Sintern 820°C/10 min (Zylinder) | | blau-weiß | weiß | schwach weiß |
| Glas nach Beispiel 14 | 30 | Glas gemäß Beispiel 31 der WO2017/055010A1 | 70 | Sintern 800°C/10 min (Zylinder) | | blau-weiß | schwach weiß | schwach weiß |
| Glas nach Beispiel 21 | 10 | Glas gemäß Beispiel 3 der EP2377831A1 | 90 | Sintern 880°C/10 min (Zylinder) | | grün-gelb | schwach weiß | schwach weiß |
| Glas nach Beispiel 21 | 10 | Glas gemäß Beispiel 3 der EP2377831A1 | 90 | Sintern 900°C/10 min (Scheibe) | | gelb-weiß | weiß | schwach weiß |
| Glas nach Beispiel 21 | 20 | Glas gemäß Beispiel 3 der EP2377831A1 | 80 | Sintern 900°C/10 min (Zylinder) | | schwach gelb-weiß | schwach gelb-weiß | leicht gelb |
| Glaskeramik nach Beispiel 1 | 1 | Glas gemäß Beispiel 9 der EP1688398A1 | 99 | Sintern 940°C/10 min (Scheibe) | | blau-weiß | | |
| Glaskeramik nach Beispiel 1 | 5 | Glas gemäß Beispiel 9 der EP1688398A1 | 95 | Sintern 940°C/10 min (Scheibe) | | leicht blau-weiß | | |
| Glaskeramik nach Beispiel 1 | 5 | Glas gemäß Beispiel 31 der WO2017/055010A1 | 95 | Sintern 900°C/10 min (Scheibe) | | blau-weiß | blau-weiß | |
| Glaskeramik nach Beispiel 1 | 5 | Glas gemäß der WO2017/032745A1 | 95 | Sintern 830°C/60 min (Zylinder) | leicht weiß | blau-weiß | blau-weiß | schwach blau-weiß |
| Glaskeramik nach Beispiel 2 | 1 | Glas gemäß der WO2017/032745A1 | 99 | Sintern 820°C/60 min (Scheibe) | | blau-weiß | schwach blau-weiß | |
| Glaskeramik nach Beispiel 2 | 5 | Glas gemäß der WO2017/032745A1 | 95 | Sintern 820°C/60 min (Scheibe) | schwach blau-weiß | stark blau-weiß | blau-weiß | leicht blau-weiß |
| Glaskeramik nach Beispiel 2 | 5 | Glas gemäß Beispiel 3 der EP2377831A1 | 95 | Heißpressen 920°C/25 min (Pressstumpf) | | stark blau-weiß | schwach blau-weiß | |
| Glaskeramik nach Beispiel 2 | 5 | Glas gemäß Beispiel 3 der EP2377831A1 | 95 | Sintern 900°C/10 min (Scheibe) | leicht weiß | stark blau-weiß | schwach blau-weiß | schwach blau-weiß |
| Glaskeramik nach Beispiel 2 | 10 | Glas gemäß Beispiel 3 der EP2377831A1 | 90 | Sintern 900°C/10 min (Scheibe) | leicht weiß | stark blau-weiß | blau-weiß | schwach blau-weiß |
| Glaskeramik nach Beispiel 2 | 10 | Glas gemäß Beispiel 3 der EP2377831A1 | 90 | Sintern 890°C/10 min (Zylinder) | blau-weiß | blau-weiß | blau-weiß | weiß |
| Glaskeramik nach Beispiel 5 | 5 | Glas gemäß Beispiel 3 der EP2377831A1 | 95 | Sintern 890°C/10 min (Zylinder) | schwach blau | stark blau | weiß | weiß |
| Glaskeramik nach Beispiel 5 | 5 | Glas gemäß Beispiel 3 der EP2377831A1 | 95 | Sintern 900°C/25 min (Zylinder) | schwach blau | stark blau-weiß | blau-weiß | schwach blau |
| Glaskeramik nach Beispiel 5 | 10 | Glas gemäß Beispiel 3 der EP2377831A1 | 90 | Sintern 900°C/10 min (Zylinder) | schwach blau | stark blau-weiß | blau-weiß | leicht weiß |
| Glaskeramik nach Beispiel 5 | 10 | Glas gemäß Beispiel 3 der EP2377831A1 | 90 | Heißpressen 920°C/25 min (Pressstumpf) | schwach blau | stark blau-weiß | blau-weiß | schwach weiß |
| Glaskeramik nach Beispiel 4 | 10 | Glas gemäß der WO2017/032745A1 | 90 | Sintern 810°C/30 min (Scheibe) | blau-weiß | stark blau-weiß | blau-weiß | blau-weiß |
| Glaskeramik nach Beispiel 9 | 10 | Glas gemäß der WO2017/032745A1 | 90 | Sintern 810°C/30 min (Scheibe) | blau-weiß | stark blau-weiß | blau-weiß | blau-weiß |

| | | | | | | | | |
|---|---|---|---|---|---|---|---|---|
| * Fluoreszenz des wärmebehandelten Körpers bei der angegebenen Anregungswellenlänge; ** Gew.-% | | | | | | | | |

## Patentansprüche

1. Glas oder Glaskeramik mit Gehalt an Europium, die die folgenden Komponenten enthalten:
| Komponente | Gew.-% |
|---|---|
| SiO₂ | 30,0 bis 75,0 |
| Al₂O₃ | 10,0 bis 45,0 |
| Europium, berechnet als Eu₂O₃ | 0,05 bis 5,0. |

2. Glas oder Glaskeramik nach Anspruch 1, die 32,0 bis 72,0, insbesondere 35,0 bis 65,0 und bevorzugt 38,0 bis 50,0 Gew.-% SiO₂ enthalten.

3. Glas oder Glaskeramik nach Anspruch 1 oder 2, die 15,0 bis 40,0, insbesondere 20,0 bis 40,0, bevorzugt 25,0 bis 40,0 und besonders bevorzugt 30,0 bis 40,0 Gew.-% Al₂O₃ enthalten.

4. Glas oder Glaskeramik nach einem der Ansprüche 1 bis 3, die 0,1 bis 4,0, insbesondere 0,3 bis 3,0, bevorzugt 0,5 bis 2,0, weiter bevorzugt 0,6 bis 1,0 und besonders bevorzugt 0,65 bis 0,85 Gew.-% Europium, berechnet als Eu₂O₃, enthalten.

5. Glas oder Glaskeramik nach einem der Ansprüche 1 bis 4, die 8,0 bis 30,0 Gew.-%, insbesondere 12,0 bis 29,0, bevorzugt 15,0 bis 28,0 und besonders bevorzugt 20,0 bis 27,0 Gew.-% Me^{II}O enthalten, wobei Me^{II}O ausgewählt ist aus MgO, CaO, SrO und/oder ZnO.

6. Glas oder Glaskeramik nach einem der Ansprüche 1 bis 5, die 8,0 bis 30,0, insbesondere 12,0 bis 29,0, bevorzugt 15,0 bis 28,0 und besonders bevorzugt 20,0 bis 27,0 Gew.-% CaO und/oder SrO enthalten.

7. Glas oder Glaskeramik nach einem der Ansprüche 1 bis 6, die mindestens eine und bevorzugt alle der folgenden Komponenten in den angegebenen Mengen enthalten:
| Komponente | Gew.-% |
|---|---|
| MgO | 0 bis 13,0, insbesondere 3,5 bis 13,0 |
| CaO | 0 bis 22,0, insbesondere 5,0 bis 22,0 |
| SrO | 0 bis 28,0, insbesondere 9,0 bis 28,0 |
| ZnO | 0 bis 5,0, insbesondere 4,0 bis 5,0. |

8. Glas oder Glaskeramik nach einem der Ansprüche 1 bis 7, die 0 bis 10,0, insbesondere 0 bis 5,0 und bevorzugt 0 bis 1,0 Gew.-% BaO enthalten und am meisten bevorzugt im Wesentlichen frei von BaO sind.

9. Glas oder Glaskeramik nach einem der Ansprüche 1 bis 8, die 0 bis 2,0, insbesondere 0,1 bis 1,2 und bevorzugt 0,3 bis 0,7 Gew.-% Zinn, berechnet als SnO, enthalten.

10. Glas oder Glaskeramik nach einem der Ansprüche 1 bis 9, die 0 bis 5,0, insbesondere 0,5 bis 4,0 und bevorzugt 1,0 bis 3,0 Gew.-% Cer, berechnet als CeO₂, enthalten.

11. Glas oder Glaskeramik nach einem der Ansprüche 1 bis 10, die 0 bis 3,0, insbesondere 0 bis 2,0 und bevorzugt 0 bis 1,0 Gew.-% B₂O₃ enthalten und am meisten bevorzugt im Wesentlichen frei von B₂O₃ sind.

12. Glas oder Glaskeramik nach einem der Ansprüche 1 bis 11, die mindestens eine und bevorzugt alle der folgenden Komponenten in den angegebenen Mengen enthalten:
| Komponente | Gew.-% |
|---|---|
| SiO₂ | 32,0 bis 72,0 |
| Al₂O₃ | 15,0 bis 40,0 |
| Europium, berechnet als Eu₂O₃ | 0,1 bis 4,0 |
| Cer, berechnet als CeO₂ | 0 bis 5,0 |
| Zinn, berechnet als SnO | 0 bis 2,0 |
| Me^{I}₂O | 0 bis 15,0 |
| Me^{II}O | 0 bis 30,0 |
| Me^{III}₂O₃ | 0 bis 10,0 |
| Me^{IV}O₂ | 0 bis 15,0 |
| Me^{V}₂O₅ | 0 bis 6,0 |
| Me^{VI}O₃ | 0 bis 6,0 |
| Fluor | 0 bis 5,0, |
wobei
Me^{I}₂O insbesondere ausgewählt ist aus Li₂O, Na₂O, K₂O, Rb₂O und/oder Cs₂O,
Me^{II}O insbesondere ausgewählt ist aus MgO, CaO, SrO und/oder ZnO,
Me^{III}₂O₃ insbesondere ausgewählt ist aus Y₂O₃, La₂O3, Ga₂O₃ und/oder In₂O₃,
Me^{IV}O₂ insbesondere ausgewählt ist aus ZrO₂ und/oder GeO₂, Me^{V}₂O₅ insbesondere ausgewählt ist aus P₂O₅, V₂O₅, Ta₂O₅ und/oder Nb₂O₅ und
Me^{VI}O₃ insbesondere ausgewählt ist aus WO₃ und/oder MoO₃.

13. Glaskeramik nach einem der Ansprüche 1 bis 12, die mindestens ein Alumosilikat, insbesondere ein Calciumalumosilikat oder Strontiumalumosilikat, als Kristallphase und vorzugsweise als Hauptkristallphase aufweist.

14. Glaskeramik nach einem der Ansprüche 1 bis 13, die mindestens 5 Gew.-%, insbesondere mindestens 10 Gew.-% und bevorzugt mindestens 20 Gew.-% Alumosilikat, insbesondere Calciumalumosilikat, Strontiumalumosilikat oder Mischungen von diesen, als Kristallphase enthält.

15. Glas nach einem der Ansprüche 1 bis 12, das Keime für die Kristallisation von einem Alumosilikat und insbesondere von Calciumalumosilikat und/oder Strontiumalumosilikat enthält.

16. Glas oder Glaskeramik, die das Glas oder die Glaskeramik mit Gehalt an Europium nach einem der Ansprüche 1 bis 15 enthalten, vorzugsweise in einer Menge von 0,1 bis 50 Gew.-%, insbesondere 0,2 bis 40 Gew.-%, bevorzugt 0,5 bis 30 Gew.-%, besonders bevorzugt 1 bis 20 Gew.-% und ganz besonders bevorzugt 5 bis 10 Gew.-%.

17. Glas oder Glaskeramik nach einem der Ansprüche 1 bis 16, wobei das Glas und die Glaskeramik in Form von einem Pulver, einem Granulat, einem Rohling oder einer dentalen Restauration vorliegen.

18. Glas oder Glaskeramik nach einem der Ansprüche 1 bis 17, die bei einer Anregungswellenlänge im Bereich von 250 nm bis 430 nm und insbesondere bei einer Anregungswellenlänge im Bereich von 360 nm bis 430 nm Fluoreszenz zeigen.

19. Verfahren zur Herstellung der Glaskeramik nach einem der Ansprüche 1 bis 14 und 16 bis 18, bei dem das Glas gemäß einem der Ansprüche 1 bis 12 und 15 bis 18 mindestens einer Wärmebehandlung bei einer Temperatur von 1000 bis 1500°C, vorzugsweise 1050 bis 1450°C, für eine Dauer von insbesondere 10 bis 720 min, vorzugsweise 30 bis 120 min, unterworfen wird.

20. Verfahren nach Anspruch 19, bei dem
(a) Pulver des Glases, gegebenenfalls nach Zugabe weiterer Komponenten, zu einem Pulverpressling gepresst wird, und
(b) der Pulverpressling einer Wärmebehandlung bei einer Temperatur von 1000 bis 1500°C, vorzugsweise 1050 bis 1450°C, für eine Dauer von insbesondere 10 bis 720 min, vorzugsweise 30 bis 120 min, unterworfen wird.

21. Verwendung des Glases oder der Glaskeramik gemäß einem der Ansprüche 1 bis 15 als Mischungskomponente zur Einstellung der Fluoreszenz eines Glases oder einer Glaskeramik.

22. Verwendung des Glases oder der Glaskeramik gemäß einem der Ansprüche 1 bis 18 als Dentalmaterial und insbesondere zur Herstellung dentaler Restaurationen.

23. Verwendung nach Anspruch 22, wobei dem Glas oder der Glaskeramik durch Verpressen, Sintern oder maschinelle Bearbeitung, insbesondere im Rahmen eines CAD/CAM-Verfahrens, die Form der gewünschten dentalen Restauration, insbesondere Inlay, Onlay, Krone, Teilkrone, Brücke, Veneer, Schale oder Abutment, gegeben wird.

24. Verfahren zur Herstellung einer dentalen Restauration, insbesondere Inlay, Onlay, Krone, Teilkrone, Brücke, Veneer, Schale oder Abutment, bei dem dem Glas oder der Glaskeramik gemäß einem der Ansprüche 1 bis 18 durch Verpressen, Sintern oder maschinelle Bearbeitung, insbesondere im Rahmen eines CAD/CAM-Verfahrens, die Form der gewünschten dentalen Restauration gegeben wird.
